**Europäisches Patentamt**

**European Patent Office**

**Office européen des brevets**

(19)

(11) Veröffentlichungsnummer : **0 370 343 B1**

(12) **EUROPÄISCHE PATENTSCHRIFT**

(45) Veröffentlichungstag der Patentschrift :
**03.02.93 Patentblatt 93/05**

(21) Anmeldenummer : **89120973.6**

(22) Anmeldetag : **11.11.89**

(51) Int. Cl.⁵ : **C07C 39/17,** C07C 37/14,
C07C 37/16

(54) **Verfahren zur Herstellung von 4-Methyl-2-cyclohexyl-phenol.**

(30) Priorität : **25.11.88 DE 3839853**

(43) Veröffentlichungstag der Anmeldung :
**30.05.90 Patentblatt 90/22**

(45) Bekanntmachung des Hinweises auf die
Patenterteilung :
**03.02.93 Patentblatt 93/05**

(84) Benannte Vertragsstaaten :
**DE FR GB IT**

(56) Entgegenhaltungen :
**DE-A- 2 437 322**
**GB-A- 731 270**
**GB-A- 802 884**
**GB-A- 998 186**
**US-A- 1 917 823**

(56) Entgegenhaltungen :
**JOURNAL OF APPLIED CHEMISTRY OF THE
USSR, Band 41, Nr. 2, Februar 1968, Seiten
381-384; F. M. EGIDIS et al: "Alkylation ofpcresol with cyclohexene and cyclohexanol in
the presence of KU-2 cation exchange resin"
BEILSTEINS HANDBUCH DER ORGANI-
SCHEN CHEMIE, 4. Auflage, 3. Ergänzungswerk, 6. Band, 1966, Seite 2531, Springer
Verlag, Berlin**

(73) Patentinhaber : **BAYER AG**
**W-5090 Leverkusen 1 Bayerwerk (DE)**

(72) Erfinder : **Klein, Alfons**
**Virchowstrasse 9**
**W-4000 Düsseldorf 1 (DE)**
Erfinder : **Fiege, Helmut Dr.**
**Walter-Flex-Strasse 23**
**W-5090 Leverkusen 1 (DE)**
Erfinder : **Puppe, Lothar, Dr.**
**Am Weiher 10a**
**W-5093 Burscheid (DE)**
Erfinder : **Jeblick, Werner, Dr.**
**Elisabeth-Langgässer-Strasse 3**
**W-5090 Leverkusen 1 (DE)**

EP 0 370 343 B1

**Beschreibung**

Die Erfindung betrifft ein Verfahren zur Herstellung von 4-Methyl-2-cyclohexyl-phenol aus p-Kresol und Cyclohexanol oder Cyclohexen.

4-Methyl-2-cyclohexyl-phenol dient zur Herstellung von Stabilisatoren für Thermoplaste und Elastomere (DE-OS 30 21 726).

4-Methyl-2-cyclohexyl-phenol kann in "mäßiger" Ausbeute (ohne nähere Angaben) durch Umsetzung von p-Kresol mit Cyclohexanol in 72 %iger Schwefelsäure bei 60° hergestellt werden (J. Prakt. Chem. N. F. 159 (1941), 155, bes. 164).

Es ist auch bekannt geworden, diese Reaktion mit Aluminiumchlorid bzw. mit Phosphorsäure zu katalysieren (Zh. Organ. Khim. 1 (1965), 510 der englischen Übersetzung). Hierbei wird in beiden Fällen bei 90-100°C gearbeitet; im Falle des AlCl$_3$ wird eine äquimolare Menge Katalysator, aber eine 5-fach molare Menge p-Kresol, bezogen auf das Cyclohexanol, gewählt, wobei der Katalysator zur Mischung der Reaktionspartner zugegeben wird. Im Falle der Phosphorsäure wird diese im 3-fach molaren Überschuß über das p-Kresol zusammen mit diesem vorgelegt und sodann eine zum p-Kresol äquimolare Menge an Cyclohexanol zugegeben. Die Ausbeuten werden mit 61 % (AlCl$_3$) bzw. 78 % (H$_3$PO$_4$) angegeben.

In Zh. Prikl. Khim. 41 (2) (1968), 381 der englischen Übersetzung wird die Umsetzung von p-Kresol mit Cyclohexen in Gegenwart von sauren Ionenaustauschern beschrieben. Hierbei wird auch bei einem Überschuß von p-Kresol stets ein beträchtlicher Anteil des 2-fach mit Cyclohexyl substituierten p-Kresols erhalten. Daneben bilden sich beträchtliche Mengen Cyclohexyl-p-tolylether. Führt man unter solchen Bedingungen die Alkylierung mit Cyclohexanol durch, ist das vorherrschende Reaktionsprodukt der Cyclohexyl-p-tolylether.

Weiterhin ist gemäß US 1.917.823 versucht worden, Phenol und o-Kresol mit Cyclohexen bzw. Cyclohexanol in Gegenwart von Bleicherden umzusetzen. Hierbei werden durchweg hohe Temperaturen und überatmosphärische Drücke angewandt. Gemäß diesem US-Patent ist die Alkylierung in der ortho-Position nicht die endgültige Substitutionsstellung; vielmehr wird unter dem Einfluß höherer Reaktionstemperaturen das ortho-Produkt in das para-Produkt umgelagert; bei anfänglichem Zusatz von o-Cyclohexyl-phenol wird sogar die weitere Bildung dieses Produktes unterdrückt.

In DE-OS 24 37 322 werden als Katalysatoren für die Alkylierung mit Hilfe von Alkenen auch bereits Molekularsiebe eingesetzt. Hierbei wird jedoch als phenolischer Reaktionspartner das m-Kresol eingesetzt, welches in einer ersten Stufe leicht zu einem Gemisch der verschiedenen isomeren Alkyl-m-kresole umgesetzt wird, die in einem zweiten Schritt unter Isomerisierung das thermodynamisch stabilste 5-Alkyl-3-methyl-phenol ergeben. Die Ausführungsbeispiele dieser DE-OS geben keine Ausbeuten an. Hingegen werden im ersten Reaktionsschritt stets unterschiedlich zusammengesetzte Gemische der theoretisch möglichen Isomeren gezeigt; dies gilt sowohl für die überwiegend mit Propen hergestellten Alkylierungsprodukte als auch für einige mit 1-Buten, Cyclohexen und Styrol hergestellten Reaktionsprodukte.

Man erkennt, daß ältere Katalysatoren, wie H$_2$SO$_4$, AlCl$_3$ und H$_3$PO$_4$, die häufig in großen Mengen angewendet werden, beträchtliche Entsorgungsprobleme bringen. Weiterhin ist erkennbar, daß unlösliche Katalysatoren, wie Ionenaustauscher, Bleicherden und Molekularsiebe zum Teil Produktverschiebungen ergeben oder nur auf spezielle Substrate erfolgreich Anwendung finden.

Es wurde nun ein Verfahren zur Herstellung von 4-Methyl-2-cyclohexyl-phenol durch Alkylierung von p-Kresol mit Cyclohexanol oder Cyclohexen gefunden, das dadurch gekennzeichnet ist, daß die Alkylierung in flüssiger Phase in Gegenwart von 1-10 Gew.-%, bevorzugt 2-4 Gew.-%, bezogen auf die Menge an p-Kresol, eines weitporigen, sauren Zeoliths durch Zugabe von Cyclohexanol bzw. Cyclohexen zum vorgelegten p-Kresol durchgeführt wird.

Das molare Verhältnis von Cyclohexanol oder Cyclohexen zu p-Kresol beträgt 1:1-4, bevorzugt 1:1,1-2, besonders bevorzugt 1:1,3-1,8.

Das erfindungsgemäße Verfahren wird bei einer Temperatur von 140-200°C, bevorzugt 150-180°C durchgeführt. Der Druck ist für das erfindungsgemäße Verfahren nicht kritisch und ist lediglich erforderlich, um den größten Teil der Reaktionspartner in der flüssigen Phase zu halten; beispielsweise wird bei 1-10 bar, in vielen Fällen bei 1-5 bar gearbeitet. Sofern bei einem überatmosphärischen Druck gearbeitet wird, ist der Eigendruck des Reaktionssystems der bevorzugte Arbeitsdruck.

Der Einsatz von Cyclohexanol im erfindungsgemäßen Verfahren ist mit der Abspaltung von Reaktionswasser verbunden. Da Molekularsiebe Wasser absorbieren können, das sie vollständig erst bei Temperaturen oberhalb 400° wieder abgeben, konnte erwartet werden, daß zumindest ein Teil der Kanäle und Poren der Zeolithe im Falle der Verwendung von Cyclohexanol durch Wasser belegt würde, wodurch die katalytische Aktivität dieser Zeolithe drastisch gesenkt würde. Erstaunlicherweise wird diese Erscheinung beim Einsatz von Cyclohexanol nicht beobachtet; darüber hinaus ist Cyclohexanol billiger, so daß der Einsatz von Cyclohexanol im erfindungsgemäßen Verfahren eine bevorzugte Variante darstellt.

Grundsätzlich ist es beim Einsatz von Cyclohexanol möglich, daß Reaktionswasser im Reaktionsgemisch zu belassen und erst bei der Aufarbeitung abzutrennen. In einer bevorzugten Variante wird das Wasser jedoch absatzweise oder kontinuierlich aus dem Reaktionsgemisch entfernt, was in einer dem Fachmann bekannten Weise auch beim Arbeiten unter erhöhtem Druck möglich ist. Auf diese Art und Weise ist der Fortschritt der Reaktion zu beobachten. Bei dieser Variante kann das Cyclohexanol im Maße der Wasserabspaltung (= Fortschritt der Alkylierungsreaktion) zum Reaktionsgemisch gegeben werden. Im Falle der Verwendung von Cyclohexen wird dies in dem Maße zum Reaktionsgemisch gegeben, daß nie ein nennenswerter Cyclohexen-Rückfluß erkennbar ist.

Zur Verfahrensdurchführung wird beispielsweise ein Gemisch aus dem Zeolith und p-Kresol unter Rühren auf die gewünschte Reaktionstemperatur erhitzt. Danach wird Cyclohexanol gemäß der entwickelten Wassermenge zugegeben. Für einen Reaktionsansatz mit 2-4 Mol p-Kresol ist dazu im allgemeinen eine Zeit von 3-6 Stunden erforderlich. Nach Beendigung der Cyclohexanolzugabe läßt man noch 2-4 Stunden bei Reaktionstemperatur nachrühren. Danach kann das 4-Methyl-2-cyclohexyl-phenol durch dem Fachmann bekannte Trennmethoden aus dem Reaktionsgemisch gewonnen werden. So ist es beispielsweise möglich, zunächst den Zeolith durch Filtration oder Zentrifugieren zu entfernen. Es ist aber auch möglich, den Zeolith nach Beendigung der Reaktion in der Wärme absitzen zu lassen und das Reaktionsgemisch abzudekantieren oder abzuhebern. Eine solche Verfahrensweise beläßt den vielfach wiederverwendbaren Kontakt im Reaktionsgefäß, worin nach erneuter Zugabe von p-Kresol und nachfolgende Zugabe von Cyclohexanol weiteres Reaktionsprodukt gewonnen werden kann. Das vom Zeolith befreite Reaktionsgemisch kann vorteilhafterweise durch Destillation weiter aufgearbeitet werden. Das dabei anfallende überschüssige p-Kresol wird wieder zurückgeführt.

Die Durchführung unter Benutzung von Cyclohexen ist grundsätzlich die gleiche; die Zugabe erfolgt, wie bereits oben erwähnt, in dem Maße, daß kein nennenswerter Cyclohexen-Rückfluß erkennbar ist.

Das erfindungsgemäße Verfahren wird in Gegenwart von weitporigen, sauren Zeolithen durchgeführt. Zeolithe sind kristalline Alumosilikate, die aus einem Netzwerk von $SiO_4$- bzw. $AlO_4$-Tetraedern aufgebaut sind. Die einzelnen Tetraeder sind mit Sauerstoffbrücken über die Ecken untereinander verknüpft und bilden ein räumliches Netzwerk, das von Kanälen und Hohlräumen durchzogen ist. Als Ausgleich für die negative Ladung des Gitters sind austauschbare Kationen eingelagert. Zeolithe können durch folgende allgemeine Formel dargestellt werden:

$$M_{m/z}[m\,AlO_2 \cdot nSiO_2] \cdot q\,H_2O \qquad (I),$$

worin

n/m das Si/Al-Verhältnis darstellt,

$M_{m/z}$ austauschbare Kationen bedeutet, wobei

z die Wertigkeit des Kations angibt und

q die Menge der sorbierten Wasserphase angibt.

Zeolithe verschiedener Struktur sind beispielsweise in D.W. Breck, Zeolite Molecular Sieves, John Wiley and Sons Inc., New York, 1974, beschrieben. In solchen Zeolithen kann weiterhin das Aluminium teilweise durch andere dreiwertige Ionen, wie z.B. Fe(III), Ga (III), B (III) u.a. ersetzt sein.

Die erfindungsgemäßen Zeolithe sind weitporig und haben Porenweiten von 7 bis 9 Å.

Die erfindungsgemäß einsetzbaren Zeolithe haben weiterhin einen sauren Charakter. Dies wird erreicht, indem beispielsweise die Metallkationen, die der Zeolith aus seinem natürlichen Ursprung oder aus seiner Synthese enthält, durch Mineralsäuren im Sinne eines Ionenaustausches ersetzt werden. Des weiteren kann ein Austausch von Metallkationen durch das Ammoniumion erfolgen; bei einer nachfolgenden Kalzinierung entweicht sodann $NH_3$ und ein protonenhaltiger Zeolith bleibt zurück. Eine weitere Möglichkeit zur sauren Einstellung von Zeolithen besteht darin, Zeolithe mit Ionen der Wertigkeitsstufe 3 (z.B. seltenen Erden, Al, Fe, Ga, In) oder der Wertigkeitsstufe 2 (wie z.B. Erdalkali- und zweiwertige Übergangsmetallkationen oder zweiwertige Hauptgruppenmetallkationen) auszutauschen (s. P.A. Jacobs, Carboionogenic Activity of Zeolites, Elsevier Scientific Publishing Company, Amsterdam, 1977). Man kann durch den Austauschgrad ($MeO/Al_2O_3$, bzw. $Me_2O_3/Al_2O_3$ ausgedrückt in Molen) unterschiedliche Aciditätsabstufungen im Zeolith einstellen.

Erfindungsgemäß einsetzbare Zeolithtypen sind beispielsweise: Zeolith Y, ZSM 12, Zeolith L, Zeolith Ω, Zeolith β, ZSM 20, Mordenit, Offretit, Cancrinit oder Gmelinit.

Bevorzugt eingesetzt werden: Zeolith Y, ZSM 20 und Zeolith L. Die Zeolithe können sowohl in der Protonenform mit Austauschgraden von 5 bis 100 % für das Proton eingesetzt werden als auch in der mit 3-wertigen Ionen ausgetauschten Form wie z.B. seltene Erden entweder einzeln oder in unterschiedlichen Gemischen in Austauschgraden von 5 bis 100 %, bevorzugt 10 bis 100 %. Ebenfalls geeignet sind Austauschformen mit weiteren 3-wertigen Kationen wie z.B. Aluminium, Eisen, Chrom, Gallium, Indium o.ä.

Weiterhin geeignet sind mit 2-wertigen Kationen ausgetauschte Formen wie z.B. mit Erdalkalimetallen, Mangan, Kobalt, Nickel, Kupfer, Zink, Cadmium, die ebenfalls acide Zentren im Zeolith erzeugen können. Durch Variation des Austauschgrades von 5 bis 100 % können die unterschiedlichen Aciditätsabstufungen eingestellt werden.

Bevorzugte Austauschformen sind die Protonenformen, wobei die Austauschgrade zwischen 50 und 100 % liegen. Eine weitere bevorzugte Austauschform umfaßt die weitporigen Zeolithe mit seltenen Erden als Kationenform, wobei die bevorzugte Aciditätsstufe durch einen Austauschgrad von 20 bis 90 % erhalten werden kann. Geeignet sind sowohl La-reiche Seltenerdgemische als auch Ce-reiche Seltenerdgemische, wie sie in der Technik eingesetzt werden. Ebenfalls eingesetzt werden können die reinen Lanthaniden wie z.B. La, Ce, Pr, Nd, Pm, Sm, Eu, Gd, u.a.

Beispiel 1

Herstellung der $H^+$-Form von Zeolith Y 100 g der $Na^+$-Form von Zeolith Y mit einem $SiO_2/Al_2O_3$-Verhältnis von 4,8 wurden mit 70g einer Ammoniumsulfat-Lösung mit einer Konzentration von 132 g $(NH_4)_2SO_4$ pro Liter gemischt. Die Suspension wurde 3 Stunden bei 90°C gerührt. Der Ionenaustausch wurde noch zweimal wiederholt. Nach dem Ionenaustausch wurde der Festkörper abfiltriert und mit Wasser zur Entfernung des überschüssigen Ammoniumsulfats gewaschen. Der Zeolith wurde 3 Stunden bei 110°C getrocknet. Um die $H^+$-Form des Zeolith Y zu erhalten, wurde das Pulver auf einem Blech mit einer Schichtdicke von 1 cm ausgebreitet. Das programmierte Erhitzen wurde mit einem Anstieg von 10°C pro Minute bis zu 420°C durchgeführt. Bei dieser Temperatur wurde der Zeolith für 1 Stunde gehalten.

Beispiel 2

In ein Gemisch von 232 g p-Kresol und 6,0 g H-Zeolith Y wurde bei 165°C innerhalb von 3 Stunden 117 g Cyclohexen eingetropft. Man ließ 2 weitere Stunden bei 165°C nachrühren und filtrierte anschließend vom Zeolith. Man erhielt 347,6 g Reaktionsgemisch mit folgender Zusammensetzung:

| | |
|---|---|
| p-Kresol: | 21,0 % |
| Unbekannte: | 0,9 % |
| Cyclohexyl-4-tolylether: | 0,2 % |
| 4-Methyl-2-cyclohexyl-phenol: | 71,7 % |
| 4-Methyl-3-cyclohexyl-phenol: | 3,8 % |
| 4-Methyl-2,6-dicyclohexyl-phenol: | 1,3 % |
| 4-Methyl-2,5-dicyclohexyl-phenol: | 1,1 % |

Danach betrug die Ausbeute an reinem 4-Methyl-2-cyclohexyl-phenol 249,2 g (92,1 % der theoretischen Ausbeute, bezogen auf eingesetztes Cyclohexen).

Beispiel 3

Zu einem Gemisch von 232 g p-Kresol und 5,0 g H-Zeolith Y ließ man bei 165°C innerhalb von 3 Stunden 142,5 g Cyclohexanol eintropfen. Das entstehende Reaktionswasser wurde laufend ausgeschleust. Man ließ 2 Stunden bei 165°C nachrühren und filtrierte anschließend vom Zeolith. Man erhielt 343,5 g Reaktionsgemisch mit folgender Zusammensetzung:

```
p-Kresol:                                        23,8 %

Unbekannte:                                       1,3 %

Cyclohexyl-4-tolylether:           .              0,1 %

4-Methyl-2-cyclohexyl-phenol:                    66,9 %

4-Methyl-3-cyclohexyl-phenol:                     4,6 %

4-Methyl-2,6-dicyclohexyl-phenol:                 2,3 %

4-Methyl-2,5-dicyclohexyl-phenol:                 1,0 %
```

Danach betrug die Ausbeute an 4-Methyl-2-cyclohexyl-phenol 229,8 g (84,9 % der theoretischen Ausbeute, bezogen auf eingesetztes Cyclohexanol).

Beispiel 4

Zu einem Gemisch von 232 g p-Kresol und 5,0 g Seltenerd-Zeolith Y (0,1 SE$_2$O$_3$/Al$_2$O$_3$) ließ man bei 165°C innerhalb von 3 Stunden 142,5 g Cyclohexanol eintropfen. Das entstehende Reaktionswasser wurde laufend ausgeschleust.

Man ließ 2 Stunden bei 165°C nachrühren und filtrierte anschließend vom Zeolith. Man erhielt 345,7 g Reaktionsgemisch mit folgender Zusammensetzung:

```
p-Kresol:                                        19,2 %

Unbekannte (4 GC-Peaks):                          1,0 %

Cyclohexyl-4-tolylether:                          0,2 %

4-Methyl-2-cyclohexyl-phenol:                    67,4 %

4-Methyl-3-cyclohexyl-phenol:                     5,1 %

4-Methyl-2,6-dicyclohexyl-phenol:                 4,4 %

4-Methyl-2,5-dicyclohexyl-phenol:                 2,7 %
```

Danach betrug die Ausbeute an 4-Methyl-2-cyclohexyl-phenol 232,6 g (85,1 % der theoretischen Ausbeute, bezogen auf eingesetztes Cyclohexanol).

**Patentansprüche**

1. Verfahren zur Herstellung von 4-Methyl-2-cyclohexyl-phenol durch Alkylierung von p-Kresol mit Cyclohexanol oder Cyclohexen, dadurch gekennzeichnet, daß die Alkylierung in flüssiger Phase in Gegenwart von 1-10 Gew.-%, bevorzugt 2-4 Gew.-%, bezogen auf die Menge an p-Kresol, eines weitporigen, sauren Zeoliths durch Zugabe von Cyclohexanol bzw. Cyclohexen zum vorgelegten p-Kresol durchgeführt wird.

2. Verfahren nach Anspruch 1, dadurch gekennzeichnet, daß ein molares Verhältnis Cyclohexanol oder Cyclohexen zu p-Kresol von 1:1-4, bevorzugt 1:1,1-2, besonders bevorzugt 1:1,3-1,8, eingestellt wird.

3. Verfahren nach Anspruch 1, dadurch gekennzeichnet, daß die Alkylierung bei 140-200°C, bevorzugt bei 150-180°C durchgeführt wird.

4. Verfahren nach Anspruch 1, dadurch gekennzeichnet, daß zur Alkylierung Cyclohexanol eingesetzt wird.

5. Verfahren nach Anspruch 4, dadurch gekennzeichnet, daß entstehendes Wasser aus dem Reaktionsgemisch entfernt wird.

6. Verfahren nach Anspruch 1, dadurch gekennzeichnet, daß als weitporige, saure Zeolithe solche mit Po-

EP 0 370 343 B1

renweiten von 7 bis 9 Å eingesetzt werden.

7. Verfahren nach Anspruch 1, dadurch gekennzeichnet, daß als saure Zeolithe solche eingesetzt werden, die zu 5 bis 100 % mit Protonen oder 2- oder 3-wertigen Metallkationen, bevorzugt mit Protonen oder 3-wertigen Metallkationen, ausgetauscht sind.

8. Verfahren nach Anspruch 7, dadurch gekennzeichnet, daß zu 10 bis 100 %, bevorzugt 50 bis 100 %, mit Protonen ausgetauschte Zeolithe eingesetzt werden.

9. Verfahren nach Anspruch 7, dadurch gekennzeichnet, daß zu 20 bis 90 % mit Kationen der Seltenerdmetalle ausgetauschte Zeolithe eingesetzt werden.

10. Verfahren nach Anspruch 9, dadurch gekennzeichnet, daß mit Kationen von Ce-reichen und/oder La-reichen Seltenerdgemischen ausgetauschte Zeolithe eingesetzt werden.

## Claims

1. Process for preparing 4-methyl-2-cyclohexylphenol by alkylation of p-cresol with cyclohexanol or cyclohexene, characterized in that the alkylation is carried out in the liquid phase in the presence of 1-10 % by weight, preferably 2-4 % by weight, based on the amount of p-cresol, of a wide-pored, acidic zeolite by adding the cyclohexanol or cyclohexene to the p-cresol initially introduced.

2. Process according to Claim 1, characterized in that a molar ratio of cyclohexanol or cyclohexene to p-cresol of 1:1-4, preferably 1:1.1-2, particularly preferably 1:1.3-1.8, is set.

3. Process according to Claim 1, characterized in that the alkylation is carried out at 140-200°C, preferably at 150-180°C.

4. Process according to Claim 1, characterized in that cyclohexanol is used for the alkylation.

5. Process according to Claim 4, characterized in that water formed is removed from the reaction mixture.

6. Process according to Claim 1, characterized in that the wide-pored, acidic zeolites used are those having pore widths of 7 to 9 Å.

7. Process according to Claim 1, characterized in that the acidic zeolites used are those which have been exchanged to an extent of 5 to 100 % with protons or 2- or 3-valent metal cations, preferably with protons or 3-valent metal cations.

8. Process according to Claim 7, characterized in that the zeolites used have been exchanged to an extent of 10 to 100 %, preferably 50 to 100 %, with protons.

9. Process according to Claim 7, characterized in that the zeolites used have been exchanged to an extent of 20 to 90 % with cations of rare earth metals.

10. Process according to Claim 9, characterized in that the zeolites used have been exchanged with cations of Ce-rich and/or La-rich rare earth mixtures.

## Revendications

1. Procédé de préparation de 4-méthyl-2-cyclohexylphénol par alkylation de p-crésol avec du cyclohexanol ou du cyclohexène, caractérisé en ce qu'on effectue l'alkylation en phase liquide en présence de 1-10 % en poids, de préférence de 2-4 % en poids par rapport à la quantité de p-crésol, d'une zéolite acide à larges pores, en ajoutant du cyclohexanol ou du cyclohexène au p-crésol introduit au préalable.

2. Procédé selon la revendication 1, caractérisé en ce qu'on établit un rapport molaire du cyclohexanol ou du cyclohexène au p-crésol de 1:1-4, de préférence de 1:1,1-2, de façon particulièrement préférée de 1:1,3-1,8.

6

3. Procédé selon la revendication 1, caractérisé en ce qu'on effectue l'alkylation à 140-200°C, de préférence à 150-180°C.

4. Procédé selon la revendication 1, caractérisé en ce qu'on utilise du cyclohexanol pour l'alkylation.

5. Procédé selon la revendication 4, caractérisé en ce qu'on élimine l'eau qui se forme du mélange réactionnel.

6. Procédé selon la revendication 1, caractérisé en qu'on utilise, comme zéolites acides à larges pores, celles qui ont une largeur de pores de 7 à 9 Å.

7. Procédé selon la revendication 1, caractérisé en ce qu'on utilise comme zéolites acides des zéolites dont les cations sont remplacés à raison de 5 à 100 % par des protons ou des cations métalliques di- ou trivalents, de préférence par des protons ou des cations métalliques trivalents.

8. Procédé selon la revendication 7, caractérisé en ce qu'on utilise des zéolites dont les cations sont remplacés à raison de 10 à 100 %, de préférence de 50 à 100 %, par des protons.

9. Procédé selon la revendication 7, caractérisé en qu'on utilise des zéolites dont les cations sont remplacés à raison de 20 à 90 % par des cations de métaux du groupe des terres rares.

10. Procédé selon la revendication 9, caractérisé en ce qu'on utilise des zéolites dont les cations sont remplacés par des cations de mélanges de terres rares riches en Ce et/ou riches en La.